# EUROPEAN PATENT SPECIFICATION

(11) **EP 3 383 361 B1**
(45) Date of publication and mention of the grant of the patent: **11.08.2021**
(21) Application number: 15807622.4
(22) Date of filing: 03.12.2015
(51) Int. Cl.: A61K 8/60, A61Q 19/00, A61P 15/00, A61P 17/00, A61P 25/00, A61P 25/28, A61P 27/02, A61P 29/00

(54) **OLIGONUCLEOTIDES IN FOOD, BEVERAGE, COSMETIC AND MEDICINAL FORMULATIONS**
OLIGONUKLEOTIDE IN NAHRUNGSMITTELN, GETRÄNKEN, KOSMETISCHEN UND MEDIZINISCHEN FORMULIERUNGEN
OLIGONUCLEOTIDES DANS DES ALIMENTS, BOISSONS ET FORMULATIONS COSMETIQUES ET MEDICINALES

(43) Date of publication of application: 10.10.2018
(73) Proprietor: DNA Essence GmbH, 6363 Fürigen (CH)
(72) Inventor: KABISCH, Johannes, 64367 Mühltal (DE); FESTEL, Gunter, 6363 Fürigen (CH)
(74) Representative: Stolmár & Partner Patentanwälte PartG mbB
(86) International application number: PCT/EP2015/078484
(87) International publication number: WO 2017/092808

(56) References cited:
- DATABASE EMBL [Online] 23 January 2009 (2009-01-23), "susfleck_PG_N_23_A11 SUSFLECK Pituitary Gland Normalized Sus scrofa cDNA clone 23_A11, mRNA sequence.", XP002759074, retrieved from EBI accession no. EM_EST:FD593996 Database accession no. FD593996
- DATABASE Geneseq [Online] 24 July 2008 (2008-07-24), "Neovison vison cDNA sequence, SEQ ID NO:4.", XP002759075, retrieved from EBI accession no. GSN:ARW82334 Database accession no. ARW82334
- V. KING ET AL: "Evolution of the Male-Determining Gene SRY Within the Cat Family Felidae", GENETICS, vol. 175, no. 4, 4 February 2007 (2007-02-04), pages 1855-1867, XP055282551, US ISSN: 0016-6731, DOI: 10.1534/genetics.106.066779 -& DATABASE EMBL [Online] 1 July 2006 (2006-07-01), "Panthera tigris sex-determining region Y protein (SRY) gene, complete cds.", XP002759089, retrieved from EBI accession no. EM_STD:DQ095160 Database accession no. DQ095160
- ZHENHUA PENG ET AL: "Transcriptome Sequencing and Analysis of the Fast Growing Shoots of Moso Bamboo (Phyllostachys edulis)", PLOS ONE, vol. 8, no. 11, 7 November 2013 (2013-11-07), page e78944, XP055282548, DOI: 10.1371/journal.pone.0078944
- RILEY P R ET AL: "Structure and expression of an ovine endometrial oxytocin receptor cDNA", JOURNAL OF MOLECULAR ENDOCRINOLOGY, SOCIETY FOR ENDOCRINOLOGY, UK, vol. 15, no. 2, 1 January 1995 (1995-01-01), pages 195-202, XP009190622, ISSN: 1479-6813
- HUI-FEN ZHAOS ET AL: "Characterization of Rat 3b-Hydroxysteroid Dehydrogenase/D5-D4 Isomerase cDNAs and Differential Tissue-specific Expression of the Corresponding mRNAs in Steroidogenic and Peripheral Tissues", THE JOURNAL OF BIOLOGICAL CHEMISTRY, vol. 266, no. 1, 5 January 1991 (1991-01-05), pages 583-593, XP055282564,
- COUSIN X ET AL: "Cloning and expression of acetycholinesterase from Bungarus fasciatus venom", JOURNAL OF BIOLOGICAL CHEMISTRY, AMERICAN SOCIETY FOR BIOCHEMISTRY AND MOLECULAR BIOLOGY, US, vol. 271, no. 25, 21 June 1996 (1996-06-21), pages 15099-15108, XP002081384, ISSN: 0021-9258, DOI: 10.1074/JBC.271.25.15099
- YUN SUNG CHO ET AL: "The tiger genome and comparative analysis with lion and snow leopard genomes", NATURE COMMUNICATIONS, vol. 4, 17 September 2013 (2013-09-17), XP055282601, DOI: 10.1038/ncomms3433
- CAROLINA R. OLIVEIRA ET AL: "Oral Vaccination Based on DNA-Chitosan Nanoparticles against Schistosoma mansoni Infection", THE SCIENTIFIC WORLD JOURNAL, vol. 164, no. 12, 1 January 2012 (2012-01-01), pages 6406-11, XP055282617, DOI: 10.1016/S0014-4894(03)00110-3
- ROY K ET AL: "Oral gene delivery with chitosan--DNA nanoparticles generates immunologic protection in a murine model of peanut allergy.", NATURE MEDICINE APR 1999, vol. 5, no. 4, April 1999 (1999-04), pages 387-391, XP002759076, ISSN: 1078-8956
- None

### SEQUENCE LISTING

<110> DNA Essence GmbH
<120> Oligonucleotides in food, beverage, cosmetic and medicinal formulations
<130> 37324-FES-P-WO
<160> 18
<170> PatentIn version 3.5
<210> 1
   <211> 543
   <212> DNA
   <213> Panthera tigris altaica
<400> 1
   atgcccttgt ccagtctgtt tgccaacgcc gtgctccggg cccagcacct gcaccagctg      60
   gccgccgaca cctacaaaga gtttgagcgg gcgtacatcc cggagggaca gaggtattcc     120
   atccagaacg cgcaggctgc cttctgcttc tcggagacca tcccggcccc cacgggcaag     180
   gacgaggccc agcagagatc cgtgagtggc caggggcgcc cggccaagga tcaggggcct     240
   ccctcgtctg caggagtcag gccctgggca gccttctccc tggggccgag ggtgtgcagg     300
   gaggcagact gggcctctcc gtccctcttc ccctttttgc aggagctgga agatggcagc      360
   ccccgggccg ggcagatcct gaagcaaacc tacgacaagt ttgacacaaa cttgcgcagt     420
   gacgacgcgc tgctcaagaa ctatgggctc ctgtcctgct tcaagaagga cctgcacaag     480
   gctgagacgt acctgcgggt catgaagtgt cgccgcttcg tggaaagcag ctgtgctttc     540
   tag                                                                   543
<210> 2
   <211> 705
   <212> DNA
   <213> Panthera tigris altaica
<400> 2
   atgttgagag tattgagcag cgatgagcac cgtgaggcgg tacagcaaca aaatatcctc 60
   gctgtggagg gaacctctag cgaactttgc acggagagtc ctacctccaa ttaccggtgc 120
   gaaaccagag gaagtggtag agaccgcggt caggaccgcg tcaaacggcc catgaacgca 180
   ttcatggtgt ggtctcgtga ccaaaggcgc aaggtggctc tagagaatcc ccaaacgcaa 240
   aactcagaga tcagcaagca gctggggtac cagtggaaaa tgcttacaga agccgaaaaa 300
   tggccgttct tcgaggaggc acagagacta caggcgctgc accgagagaa atacccgggc 360
   tataaatacc gacctcgtcg gaaggccacg ccagaaaaaa gtgacaaatt gctccccgca 420
   gagccctcgt ccacactctg tagtcagctg cacgcgggag agaggttgta cgccttcccc 480
   tacaaggacg gctgtacgaa ggctgcacac tcacgaatga aggaccagtt atactcctcc 540
   tcggaaccca tgagcataac caactcgctg ctggaaccgg gacctcacag gacctccaca 600
   accctccaaa acagtcctga agacttggct ctgcagctct ccgcggatgc tcccctttac 660
   cgtaacttag agctgggagt ttctgaggct tactttgcat ggtga 705
<210> 3
   <211> 909
   <212> DNA
   <213> Panthera tigris altaica
<400> 3
   atgggggtag ctctggagca gttcttcatt tttgtagggc tgctggcgtg cctggcgtac        60
   ctggtgaagt gtgtgagatt ctccaaatgt atttttctgc acttctggaa agttttgcca       120
   aggtctttct tgaagtcaat gggacaatgg gcagtgatca ctggagcggg agatgggatt       180
   gggaaagctt attcatttga gctagcaaga caaggactca atgttgtgct tatcagccgg       240
   acactgaaaa aactacaggc cattgctgcg gagattgagt gtaccacagg gagtagcgtg       300
   aagattatac aagcagattt taccaaagac aacatctacg agcatattag agaaaaactt       360
   gaaggcttag aaattggaat tttagtcaac aatgtcggaa tgcttccaaa ctttctcccg       420
   agccacttcc ttgatacacc agatgaaatc cagagtctca tccactgtaa catcacctca       480
   gtagtgaaga tgacacagct aattctgaaa cacatggaat caaggaggaa agggctcatc       540
   ttgaacgttt ctgccggggt ggccttcttt ccctggccac tctactccac gtattcagct       600
   tccaaggctt ttgtgtgtac gttttccaaa gccctgcaag cagaatataa aaggaaagga       660
   atcattatcc aggtgctgac cccctatgct gtttcaaccc caatgacaaa gtatctaaac       720
   accaacatgg taaccaagac tgctgatgag tttgttaaag aatcactgaa ttatgtcatg       780
   attggagacg aaacctgtgg ctgcctcatc catgaaatct tgcggagctt cctgaacctg       840
   atcccatcct gggttttgta cagcagcaca ttccagaaga aatacatgga ctacctcaag       900
   cacaatacc                                                               909
<210> 4
   <211> 1620
   <212> DNA
   <213> Buceros rhinoceros silvestris
<400> 4
   atgtctcgcc agtgcgctgc aaggacccag agcaaaaccg gcttcagtgc tgcttctgcc        60
   ttcattccga acgccagcag caccagcttc tgtgtgcgtc ctgcacccca aggcggaagc       120
   tgcagtattg cccctgggta tggaaggttt actggaggtt ttggaagcag gagcctctac       180
   agttttggtg gatgcaagag gatgtctgta gctggaagag gaggtggctt ctatggacct       240
   gcaggttttg gtgctgggat ctcctgtggt ttcggtggtg cagttggtgg tgccttcgaa       300
   tttggtggtg gtgtgggtgg ccctggattc cctgctgtcc cagctggggg catccgtgaa     360
   gtctcagtca atcagagcct tctgaaacct ctcaacctgg agattgaccc caacatccag     420
   agtatccgca aggatgagaa ggatcagatt caaaccctca acaataaatt tgcctccttc     480
   atcgacaagg tccgattcct tgagcaacaa aacaaggtcc tggagaccaa gtgggccctt     540
   ctacaagaac aggggaacaa aacggtcaga aacgacatcg agcccctctt tgagacctac     600
   atcaacaacc tcaggaggca gctgagcagc ttgctgactg acaaggagaa cctggcagga     660
   gagctgagca aggtgcaaag ccttgctgag gacttcaaga acaaatacga agaggagatc     720
   aacaggcact cagctgttga gaacgagttt gtggtcctga agaaggaggt ggacgctgcc     780
   tacatgaata agacggagct gcaagccagg ctggagtccc tcatggagga gatagatttc     840
   ctcagagccc tctatgaagc cgagctgtcc cagctgcaga cccagatctc cgacacctcc     900
   gtcatcctca ccatggacaa caaccgcagc ctggacatgg acagcatcat tgcggaggtc     960
   aaagcacagt acgaggacat cgccaaccgg agccgggcag aggctgagtc ctggtaccag    1020
   tccaggtacg aagagctgca ggccacggct ggcaggcacg gggacgacct ccgcaacacc    1080
   aagcaggaga tctctgagct caaccgccac gtccagcggc tgaggtctga aatcgacagc    1140
   gtgaagaaac agtgtgccag tttgcagacg gccatcgcgg atgccgagca gcgtggggag    1200
   ctggctctca aggatgccag ggccaaactg gctgagctgg agacagccct gcagcaggcc    1260
   aagacagatc tggcccggca gctccgcgag taccaggagc tcatgaacgt caagctggcc    1320
   ctggacatcg agatcgcaac ctacaggaag ctgctggagg gcgaggagtg caggctctct    1380
   ggagaaggtg ccaatgcagt gaatatctct gtgaccagaa cctctgtagg aacaggatat    1440
   gggagtggaa actgcctcag cttcggaggc agcagtggcg ttggaggtgg ggtctgtgcc    1500
   ggaggaacgg gcttcagctc tggaagcgga caaggcacag ccgggtcatg cgtggtgggt    1560
   ggaaacagct ccagtgtgaa gtatgtctcc accacctcct ccaccaagag atgctattag    1620
<210> 5
   <211> 2175
   <212> DNA
   <213> Callorhynchus milii
<400> 5
   atggaagtgg ctattttaag cagagttgat caactagttg atgagaaact aaaggcacat     60
   ttacctaagg tacgactggt ccgcgatgct ccgagcaact gcttgtgtcc tccaggtccc    120
   ccaggagaaa aaggaaagca cggtcgaaga ggtattccag gaccatctgg accgccagga    180
   agagatggat ttccgggaaa caaaggagca ttgggtctac cagggagaag gggtctaaag     240
   ggggaacctg gtgaaaaggg agctccagga gatccaggca tctctattat tggacctcgt    300
   ggccctccag gtcaaccagg agctagaggc tttccaggat ttccgggccc gatagggttg    360
   gatggcaaaa atggcaagat gggtccaaaa ggtgaaatgg gttcaccagg ttcaagaggt 420
   gaaaagggtg aaactggtca aaagggagaa aagggtcatc gtggagaatc tggattgcat 480
   tttggtctgc atggtcaaaa gggtgagaag ggctcgcgtg gagattctgg actttcattt 540
   acacctcctg gattgtctgt gctgctagca gctgacgagc agtatatcaa acgccatcac 600
   cacttcaagg gtgaaccggg acaagctgat aataactgtt ctccagggcc tccaggtcca 660
   ccagggccct ccggacccaa aggacctcca gggtattcag gacatccagg ggcaaacgga 720
   ttaccaggga aaactggaga agctggaaaa ccaggaagag atggaatgag gggcttaact 780
   ggaatgactg gtatgaaggg tgattccgga cttccaggac gtccaggtga aaagggtgat 840
   ccaggtgctt ctggaaagtc gggagcaaag ggtgatccgg gaatgccagg gctcccagga 900
   agaaagggag agtttggtga gccagggctg cctggtccaa caggccttcc tggacctgta 960
   gggccgaagg gagataaagg aaacattgga caaaagggag agataggaag gcctggcgtt 1020
   ccaggaaagc ctggagctaa gggcatacca agttcatctg cttctgggtt aaagggagag 1080
   ccaggggaga ttggacctcc gggagaaaag ggtgaaccag gtcttcgtgg tcttcgaggg 1140
   aggagaggtc ttaggggtga aaatgggatt ccaggtgaag ctggaatgct tggagaaagg 1200
   ggtgaaaaag gagatgcagg aatatcaacc aacggaatta aaggggaacc aggttcaact 1260
   ggatctcgtg gagctccagg gcaaaagggg gaccctggcc agcctggacc aaggggttcg 1320
   acaggtcaga agggtgaagc aggaaaaact gaaattatcg attacaatgg taacatcagc 1380
   gaagccctcc agggtccccc aggacttccc ggacagccag gccaaaaggg tgaaataggt 1440
   ttgccaggta catcaggact tgatggagaa aagggtcctc gcggtaaacc aggagaggtc 1500
   ggattgcccg gtccagaagg cccaacgggc atcgaagggc taaaaggaga gccaggaatt 1560
   atgggattcc caggacctaa aggaaacaag ggagaagttg gccaggcagg ttttccgggt 1620
   ctggatggac ccacgggtga aaagggagaa agaggggaac ctggattgca aggtccacct 1680
   ggcttgccgg gacctgttgg tcttcctgga acacctggag atagaggaga aaagggtgat 1740
   ggggttgttg gtccagaagg gccacttggt ccacagggac caccaggccc aattggactc 1800
   caggggcttt caggagcaaa gggtgattta ggatcggttg gtcttaaagg tgacagaggt 1860
   cctttgggat tgcctggtgt accaggccca gcaggttcac aagggccagc aggaccaaaa 1920
   ggagaaaggg gtgataaagg agatgctgga cctccaggac caattggacc acagggaata 1980
   actggatcac taggaccaaa aggagaaaaa ggaggccggg gggagaaggg aaagagaggt 2040
   tctaaagggc ctaaagggga taagggagac caaggagtgc ctggattaga tgcaccttgc 2100
   ccattgggtg aagatggctt acctgtacaa ggctgttgga ataaaggtca gacaccatgg 2160
   ctaaatgcca aatag 2175
<210> 6
   <211> 1122
   <212> DNA
   <213> Rattus norvegicus
<400> 6
   atgcctggat ggagctgcct ggtgacagga gcaggagggt ttgtgggcca gaggatcatc      60
   cggatgttgg tgcaggagaa agaactgcag gaggtcaggg ctctggacaa agtcttcaga     120
   ccagaaacca aggaggaatt ctctaagctg cagacaaagg ccaaggtgac aatgttagaa     180
   ggagatattc tggatgccca gtacctgagg agagcgtgcc agggcatctc tgttgtcatc     240
   cacacggctt ctgtcatgga tttctcacgt gtcctaccca ggcagaccat cctagatgtc     300
   aatctgaaag gtacccagaa cctattggag gccggtatcc acgctagtgt gccagccttc     360
   atctactgca gcacagttga cgttgcaggg cccaactcct acaagaagac catcctgaat     420
   ggccgtgagg aagagcatca tgaaagcaca tggtcaaatc catacccata cagcaaaaag     480
   atggccgaga aggcagtgct ggcagccaat gggagcatcc tgaaaaatgg tggcacactg     540
   catacttgtg ccttaaggcc catgtacatt tatggggaga gaggtcaatt cctttctaga     600
   ataataatta tggccctcaa aaataagggt gttctgaacg ttactggcaa attctccata     660
   gtcaatccag tgtatgtagg caatgtggcc tgggcacata ttctggctgc caggggcctt     720
   cgagacccca agaagtcaca aaatatccaa ggacagttct actatatctc agatgacacc     780
   cctcaccaaa gctatgatga tttaaattgc accctgagca aggaatgggg cctccgcctt     840
   gattccagct ggagccttcc tctgcccctg ctctactggc ttgccttcct gctggaaact     900
   gtgagcttcc tgctgcgtcc attttacaac tataggccac cctttaactg ccacttggtc     960
   acactgtcaa atagcaagtt cactttctcc tacaagaaag ctcagagaga tctgggctat    1020
   gagccacttg tcagctggga ggaagccaag cagaaaacct cggagtggat cgggacacta    1080
   gtggagcagc acagggagac actggacaca aagtctcagt ga                       1122
<210> 7
   <211> 1483
   <212> DNA
   <213> Ovis aries
<400> 7
   ggtcatggag ggcgcgtttg cggcaaactg gagcgcggag gcggtcaacg ggagcgcggc      60
   gcctccggga accgagggca atcgcactgc cgggccgcca cagcgcaacg aggccctggc     120
   gcgggtggag gtggccgtgc tgtccctcat cctgttcctg gcgctgagcg gcaacgcgtg     180
   cgtgctgctg gcgctgcgca ccacgcgcca caagcactcg cgcctcttct tcttcatgaa     240
   gcacctgagc atagccgacc tggcggtggc ggtgttccag gtgctgccgc agcttctgtg     300
   ggacatcacg ttccgtttct acgggcccga cctgctctgc cgcctcgtca agtacctgca     360
   ggtggtgggc atgttcgcgt ccacctacct gctgctgctc atgtcgctcg accgctgcct     420
   ggccatctgc cagccgctgc gctcgctgcg ccgccgcacc gaccgcctgg cggtactcgc     480
   cacctggctc ggctgcctgg tggccagcgc gccgcaggtg cacatcttct cgctgcgcga     540
   ggtggccgac ggcgtcttcg actgctgggc cgttttcatt cagccctggg ggcccaaggc     600
   ctacatcacg tggatcacgc tcgccgtcta cattgtgccg gtcatcgtgc ttgccgcctg     660
   ctacggcctt atcagcttca agatctggca gaacttacgg ctcaagacgg aggcggcggc     720
   ggcagaggct tccgcggggg ctgagggcgc ggcggcagac tgcgcggggc gcgcggctct     780
   ggcccgcgtc agcaacgtca agctcatctc taaggctaag atccgcacgg tcaagatgac     840
   cttcatcgtc gtgctggcct tcatcgtgtg ctggacgcca ttctttttca agcagatgtg     900
   gagtgtctgg gatgccgatg cgcccaagga agcctcggct ttcatcatcg ccatgcttct     960
   ggccagcctc aacagctgct gcaacccctg gatctacatg ctcttcacgg gccacctctt    1020
   ccaagacctt gtgcagcgct tcctctgctg ctcattccgc cgcctgaaag gcagccagct    1080
   gggggagacg agcgtcacga aaaagatcca ttcgtacacc tttgtcctga gtcggcacag    1140
   ctccagccag agaagctgct cgcagccatc cacggtgtga ctgatcggcc aggggccctc    1200
   cttggcttcg actgtgcgac agcagacagt ctggctcttg gtggctgtgt acatgtgtgt    1260
   acaaggtacc ttccagtttg taccccctgc accttgggca gctgagttgg gtgggaagca    1320
   acccacggga agatggtagg gtgactcaac catcaagcca agccccagat cttctgtggg    1380
   ccccatgggt actcctgccc tgaccccact gctgccttcc tttaacagta agtcccctac    1440
   ataagaatga gttctgttct gagaaaaaaa aaaaaaaaaa aaa                      1483
<210> 8
   <211> 1818
   <212> DNA
   <213> Ape
<400> 8
   atgagctggg gcacggagct gtgggatcag ttcgacagct tagacaagca tacacaatgg      60
   ggaattgact tcttggaaag atatgccaaa tttgttaaag agaggataga aattgaacag     120
   aactatgcga aacaattgag aaatctggtt aagaagtact gccccaaacg ttcatccaaa     180
   gatgaagagc cacggtttac ctcgtgtgta gcctttttta atatccttaa tgagttaaat     240
   gactatgcag gacagcgaga agttgtagca gaagaaatgg cgcacagagt gtatggtgaa     300
   ttaatgagat atgctcatga tctgaaaact gaaagaaaaa tgcatctgca agaaggacga     360
   aaagctcaac aatatcttga catgtgctgg aaacagatgg ataatagtaa aaagaagttt     420
   gaaagagaat gtagagaggc agaaaaggca caacagagtt atgaaagatt ggataatgat     480
   actaatgcaa ccaaggcaga tgttgaaaag gccaaacagc agttgaatct gcgtacgcat     540
   atggccgatg aaaataaaaa tgaatatgct gcacaattac aaaactttaa tggagaacaa      600
   cataaacatt tttatgtagt gattcctcag atttacaagc aactacaaga aatggacgaa      660
   cgaaggacta ttaaactcag tgagtgttac agaggatttg ctgactcaga acgcaaagtt      720
   attcctatca tttcaaaatg tttggaagga atgattcttg cagcaaaatc agttgatgaa      780
   agaagagact ctcaaatggt ggtagactcc ttcaaatctg gttttgaacc tccaggagac      840
   tttccatttg aagattacag tcaacatata tatagaacca tttctgatgg gactatcagt      900
   gcatccaaac aggagagtgg gaagatggat gccaaaacca cagtaggaaa ggccaagggc      960
   aaattgtggc tctttggaaa gaagccaaag ccacagtccc cacccttaac ccctactagt     1020
   ttattcacat ccagtactcc taatgggtcc cagtttctca cattctccat tgagcccgtg     1080
   cattattgta tgaatgaaat aaaaacaggg aagcccagaa ttccttcttt cagaagcctc     1140
   aaaagagggt ggtcggtgaa gatgggccca gcactagaag atttcagtca tctgccacca     1200
   gaacagagac gtaaaaaact acagcagcgc attgatgaac ttaacagaga actacagaaa     1260
   gaatcagacc aaaaagatgc actcaacaaa atgaaagatg tatatgagaa gaatccacaa     1320
   atgggggatc cagggagttt gcagcctaaa ttagcagaga ccatgaataa cattgaccgc     1380
   ctacgaatgg aaatccataa gaatgaggct tggctctctg aagtcgaagg caaaacaggt     1440
   gggagaggag acagaagaca tagcagtgac ataaatcatc ttgtaacaca gggacgagaa     1500
   agtcctgagg gaagttacac tgatgatgca aaccaggaag tccgtgggcc accccagcag     1560
   catggtcacc acaatgagtt tgatgatgaa tttgaggatg atgatccctt gcctgctatt     1620
   ggacactgca aagctatcta cccttttgat ggacataatg aaggtactct agcaatgaaa     1680
   gaaggtgaag ttctctacat tatagaggag gacaaaggtg acggatggac aagagctcgg     1740
   agacagaacg gtgaagaagg ctacgttccc acgtcataca tagatgtaac tctagagaaa     1800
   aacagtaaag gttcctga                                                  1818
<210> 9
   <211> 378
   <212> DNA
   <213> Sus scrofa
<400> 9
   atggccggac ccagccttgc ctgctgcctg ctcggcctcc tggcgctgac ctccgcctgc    60
   tacatccaga actgccccct gggcggcaag agggccgtgc tggacctcga cgtgcgcaag   120
   tgcctcccct gcggccccgg gggcaaaggc cgctgcttcg ggcccagcat ctgctgcggg   180
   gatgagctgg gctgcttcgt gggcacagcc gaggcgctgc gctgccagga ggagaactac   240
   ctgccgtcgc cctgccagtc cggtcagaaa ccttgcggga gcgagggccg ctgcgccgcc   300
   gccggcatct gctgcaaccc tgacggctgc cgcttcgacc ccgcctgcga ccccgaagcc   360
   accttttccc agcgctga                                                  378
<210> 10
   <211> 3123
   <212> DNA
   <213> Gallus gallus
<400> 10
   atgagggtat caataccagc tgtggcagtg tgggggagaa ttgctccctc tcacagcatc      60
   actgccatta tgattacaga tgaccagcag actattgtta caggaagtca agaggggcaa     120
   atatgtctct gggatctttc atcagaatta aagatttcat ctaaagaaat tctctttggc     180
   catactgctt ctgtaacttg tttggcaaaa gcaagagagt ttgagaaaca gccatatgta     240
   gtaagtgcta ctgaaaatgg ggagatgtgt gtttggaatg tcactagtgg actgtgcatt     300
   gagaacacaa agcttcctta taggcataca gcaatctatt attaccattg ctcgttccga     360
   atgacgggtg aaggctggct tctgtgtttt ggagaatatc aggatgttct tattattgat     420
   gctaagactt tgggtgttct gcacactttg tcttctcagt cttctgattg gataaattgt     480
   atgtgtattg ttcactctgc aagaattcaa gaagactcat tggttgctgt gtctgtaact     540
   ggagttctta aagcatggga cctatcttca tctttgacca gcatacagga gaggcaaaat     600
   gtgtgtgaaa aggaatcaaa gtctctgggc tgtacaaact gccgagcagt acggttttgc     660
   acttacacag aaagactcct tctggttgtg tcttccatat gctggcaggt ctatgattat     720
   tgtgatttct ccttgctgtg tactgagttc tgtaagagtg gtcagtgctt tgctggtgga     780
   gaagtactag cagcttacag gcttatcatc tggacagaag atggttacag ttacatctac     840
   cagctgctaa acagtggact ttccaaaagc atatatcctg cagatgggaa ggtgctgaaa     900
   gaaactgttt atcctcattt actctgcttt actgatgtga aggaaaataa gagtttccct     960
   tttgtcatgg ggtttatgaa tgaaaggaag gagcctttct ataagattct tttttctggt    1020
   gaagcttcag gaagaatcac tctgtggcat gttcctgatg ttcctgcatc aacatttgat    1080
   ggttcgccac aagaaatccc aatttcaaca acatggacac ttcaggataa ttttaataaa    1140
   gcccattcaa tggcagaggg cattatagat catctttgtg attctaaaga tggaattgaa    1200
   aatgcagttg tgacttcttc cgtgtacata cccagtctgg ataagctcgt gtgtggatgt    1260
   gaaaatgggg aaattttcat aacgccaggt ctagaaactg caagagcaag acttctagaa    1320
   aatgtgtctt tgaaaggtaa tctacctcat aaagttttga atggtcatag tagcagagtt    1380
   acatgtttac tttatccaca tggtaattca gtaaggtttg atcccagctg gctgttatca    1440
   gggggccagg attctactgt gatctgttgg gatatattta ctggaaaaat cttgcaccga    1500
   tttagtctgc aatctgggcc agtgacagag ctattgagat ctccagaaaa ctgcaaattg    1560
   cagtatcata gcgtagtgtg ctgcgtgtgc agtgatcact cagtagcagt tctacacctt    1620
   cagaagaaag tttgtctctt acatgccaga aagcatctgt ttcctgtgaa gaagataaaa      1680
   tttgacccca tggaaggtct gctaattatt gggtgtgaag atgattcggt ttatgtttgg      1740
   gaaattgaaa caggcacact ggaacgacat gaaacaggtg aaatagcaaa agcaatcctt      1800
   tcttccattg gagattcaga atacttagtg gtggattctc tacatcctgt atctcaggag      1860
   tcaaaaagaa ataaaaatgt tcaatacaaa tcctccagct cttataagcg tggtatgatc      1920
   tcttacaatc ttactcagac agaaaaatct tcagataagg taatggataa cagccttatt      1980
   cagcaaccct ttactgtttt accagttaag ccaaaatgga acgatgcaaa ctttcatgtt      2040
   ctcttatttg atctggaaaa acttgtggat cttctgctgt cgtatcaacc ctgtggatta      2100
   aagtcatcaa actcattcca caactatgac actctagaaa gagccaaaag tactactgag      2160
   aaaagagcgc tgacattaaa aagaaataaa actgctgggt tcacattgtc agtggatgga      2220
   caagtagata ctgtttatgc aaaccaagtc aatagagatt ctaacacaat taggcctttg      2280
   gaagaaagtg gtggcataaa aaggcagaaa aaaatgaaga gttcaagaaa gactaaaatg      2340
   cagccatcag gaaacattga tgtgaacatc accacagata cagctaaact gcttttgtca      2400
   tgtctcttac cctggggtgt agataaagaa atagatgatc tttgtgttgg gcaattagat      2460
   atcttaaggc tgcagtgtcc ggttgctttt ggacttgtgt caaatgaacg ccacctctca      2520
   ctgatgttgc cgggatggaa atgtactgat ggtggtgtgc tagaagagca cactttaagc      2580
   aacttgtttt caaaaagagt gcttgattta tcaaacaagt atcttgctgc aactgaagga      2640
   cgtactggtg agagagatgg aacagtggat aatgtttatg gaacaaaggg tgcagaaata      2700
   atttttttct tattaagcag aatagtttta atcaacagag tttttaacat gccttcagca      2760
   gatacgagtc aaattgaaag cctacagcaa ccagaatctg ttcacaacaa gtggcaaaat      2820
   atggaagctg ccatatcttc atttcccagt ttttatggag agttaccacg caataaaagt      2880
   agacgacatt cttctgattt tgggaatgct tcactgctga aactcatttg ctgttggagt      2940
   gaccaatcta taaagattat tgaggcaatg caagcagtgc tgttggcaga agttcagagg      3000
   actatgacaa ccttgagaaa gacagcagtc tgcagggagc cattggctac agtagagaat      3060
   ggaaatggta gctgtgactt atattgtctt ggacagttgg gaagccagac caatctgcca      3120
   ttt                                                                    3123
<210> 11
   <211> 669
   <212> DNA
   <213> Varanus comodovarensis
<400> 11
   agttgcatga aagctgcccc aatgaaagaa gttagcctca gaggacaagg cagcttggct        60
   tatccaggtc tccggacaca gggaaatctg gagagcatgg gcgggcccaa tgatgccacg       120
   cgagggttga catctttggc agacactttt gagcatgtca ttgaggagct tctggacgag       180
   caggaggtca tccagcccag caaggaaaac aaggatgctg acatgtactc gtctcgggtt       240
   atgctaagca gccaagtgcc tttggagcct ccactgctct ttctgcttga ggagtacaaa       300
   aactacttgg atgctgcaaa catgtccatg agggtccggc gccactctga ccccgctcgc       360
   cgtggggagc tgagcgtgtg tgacagtatc agcgagtggg taacagcggc ggacaaaaag       420
   actgcagtgg acatgtcagg ggcaacggtg acagtcctgg aaaaagtccc agtacccaaa       480
   ggtcaactga agcaatattt ttacgagacc aaatgcaact cgaagggtta tacgaaagag       540
   ggctgcaggg gcatagacaa gaggtactgg aactcccagt gccgaactac ccagtcttat       600
   gtgagagctc tcaccatgga taataaaaag agagttggat ggcgctttat aagaatagac       660
   acttcctgt                                                                669
<210> 12
   <211> 1938
   <212> DNA
   <213> Oryctolagus cuniculus
<400> 12
   atgtccgagc gcaaagaagg cagaggcaag gggaaaggca agaaaaagga cagaggctct      60
   ggcaagaagc ccgcgcccgc cgacggcggc ccgagcccag ctttgcctcc ccgactgaaa     120
   gagatgaaaa gccaggagtc agctgcaggc tccaagctgg tgcttcgctg tgagaccagt     180
   tcagagtact cctctctcag gttcaagtgg ttcaagaatg ggaatgaatt aaaccgaaaa     240
   aacaagccac aaaacatcaa gattcaaaaa aagccaggga aatcagaact tcgcatcaac     300
   aaagcatcac tggctgattc tggagaatat atgtgcaaag tgatcagcaa attaggaaac     360
   gacagtgcct ctgccaacat caccattgtg gaatccaatg agatcatcac tggcatgcca     420
   gcctcaacag aaagagcata tgtatcttca gagtctccca ttagaatatc agtatcaaca     480
   gaaggagcaa atacttcttc atccacatcg acgtccacca ccgggacaag tcaccttgtc     540
   aagtgtgccg agaaggagaa aactttctgt gtgaatgggg gcgagtgctt catggtgaaa     600
   gacctttcaa atccttcacg atacctgtgc aagtgcccaa atgagtttac tggtgatcgc     660
   tgccaaaact acgtaatggc cagcttctac aagcatcttg ggattgaatt tatggaagca     720
   gaagagctct accagaagag ggtgctgacc ataactggca tctgcatcgc cctgctggtg     780
   gtcggcatca tgtgtgtggt ggcctactgc aaaaccaaga aacagcggaa aaagcttcat     840
   gaccggcttc ggcagagtct tcggtcagaa cggaacaaca tggtgaacat agcaaacggg     900
   cctcaccacc ccaacccacc cccagagaac gtgcagctgg tgaatcaata tgtgtctaaa     960
   aatgtcatct ccagtgagca cattgttgag agagaagcag agacatcttt ttccaccagt    1020
   cactatacct cgacagctca tcattccacc actgtcaccc agactcctag tcacagctgg    1080
   agcaatggac acactgagag catcatttcg gaaagccact ctgtaatcat gatgtcatca 1140
   gtagaaaaca gtaggcacag cagcccaaca gggggcccaa gaggacgtct taatggcttg 1200
   ggaggccctc gagagtgtaa cagcttcctc aggcatgcca gagaaacccc tgactcctac 1260
   cgagactctc ctcatagcga aaggtatgtg tcagccatga ccaccccggc tcgtatgtca 1320
   cctgtagatt tccacacgcc aagctccccc aaatcgcccc cttcggaaat gtctccgcct 1380
   gtgtccagca tgacggtgtc catgccctcc atggcagtca gccccttcgt ggaagaagag 1440
   agacccctgc ttcttgtgac tccaccaagg ctccgggaga agaagtatga ccaccatcct 1500
   cagcaattca actccttcca ccacaacccc gcgcatgaga gtaacagcct tccccccagc 1560
   cccttgagga tagtggagga tgaggagtac gaaacgaccc aggagtatga gccagctcag 1620
   gagcctgtta agaaactcgc taatagccgg cgagccaaaa gaaccaagcc caatggccac 1680
   attgccaaca ggtcggagat ggacagcaac acaagctctg agaacagtga ctcagagagt 1740
   gaaacagagg atgaaagagt aggtgaagat acgcctttcc tgggcataca gaaccccctg 1800
   gccgccagtc ttgaggcagt acctgccttc cgcctggctg acagcaggac taacccagca 1860
   ggccgcttct caacgcagga agaaatccag gccaggctgt ctagtgtaat tgctaaccaa 1920
   gaccctattg ctgtataa 1938
<210> 13
   <211> 1746
   <212> DNA
   <213> Snake
<400> 13
   atgccctcct gccagcctgg caagatgcct gcgccgtggc cttggtggct ccagctcctc 60
   ctttgcatcc cgtcctgtgt agcggttttg cccggccggg caggcgagct gaaagtgtcc 120
   acccagactg gctcggtgcg tggcctgagc ctgccggttc tcgacggcca cgtctcggcc 180
   ttcctaggca tcccctttgc ggagcctccc ctgggcagga tgcgcttcct gcgccctgag 240
   cccgtcaagc cctggcagca cgtcctggac gccacttcct acaagcccgc ctgctaccag 300
   atggtggaca ccagctaccc aggcttccag ggcacggaga tgtggaaccc caaccggggc 360
   atgagcgagg actgcctcta cctcaacatc tgggtgccct ccccgcgccc caaggatgcc 420
   cccgtcctgg tctggatcta cggagggggc ttctacagcg gcgctgcctc cctggacgtc 480
   tatgacggcc gcttcctgac ctacacgcag aacgtcatcc tggtctccct cagctaccgt 540
   gtgggggcct tcggcttttt gggcctcccg ggaagtccag aggccccggg aaacatgggg 600
   ctgctggacc agcgcttggc cctgcagtgg atccagaaca acatccatcc ttttgggggc 660
   aatccgaggg cggtcaccgt ctttggggag agtgccgggg ccgcttcggt gggcatgcac 720
   ctgctttcca cccagagccg caccctcttc cagcgggcca ttctgcagag cggcggcccc 780
   aacgcaccgt gggccaccgt cacgcctgcc gaaagccggg gccgggcggc ccttctggga 840
   aagcagctgg gctgccactt caacaacgac tccgagctgg tcagttgctt gcgctcgaaa 900
   aacccccagg agttgattga cgaggagtgg tccgtcctgc cctacaagag cattttccgc   960
   ttccccttcg tcccggtcat tgacggggac ttctttccag ataccccgga ggccatgctg  1020
   agctcgggca acttcaagga gacccaggtg ttgctgggcg tggtgaagga cgagggctcc 1080
   tacttcctga tctacgggct gcctggcttc agcaaggaca acgagagcct catcagccgg 1140
   gccgacttcc tggaaggggt gcggatgagc gtgccccacg ccaacgacat cgccactgat 1200
   gccgtggtgc tgcaatacac tgactggcag gaccaggaca acagggagaa gaaccgggag 1260
   gccctggacg acatcgtggg agaccacaac gtcatctgcc cggtcgtgca gttcgccaac 1320
   gactacgcca agcgcaacag caaggtgtac gcctacctgt ttgaccaccg ggcctccaac 1380
   ctcctctggc ccccctggat gggggtcccc catggctacg agatcgagtt tgtctttgga 1440
   ttgccgctca acgacagcct gaactacacg ccccaagaga aggagctgag ccgcagaatg 1500
   atgcgctact gggccaactt cgcccggaca gggaacccca ccgaccctgc cgataagagc 1560
   ggggcctggc ccacctacac cgcctcccag ccgcagtatg tccagctcaa cacccagccg 1620
   ctggccaccc aacccagcct gcgggcccag atctgcgcct tctggaacca cttcctcccc 1680
   aaactgctga acgccacagt cgatcccccg cgcgccgatc gcagacgccg ttccgcccgg 1740
   gcctaa 1746
<210> 14
   <211> 1155
   <212> DNA
   <213> Canis canis
<400> 14
   atggagcgcg cgctggcggc caactggagc gcggaggcgg gcaacggcag cgaggcggcc      60
   ccggcggcgc agggcaaccg cacggccggg cccccgcagc gcaacgaggc cctggcgcgc     120
   gtggaggtgg ccgtgctgtg cctcatcctc ttcctggcgc tgagcggcaa cgcgtgcgtg     180
   ctcctggcgc tgcgcaccac gcgccacaag cactcgcgcc tcttcttctt catgaagcac     240
   ctgagcatag ccgacctggt ggtggcggtg ttccaggtgc tgccgcagct gctgtgggac     300
   atcaccttcc gcttctacgg gcccgacctg ctgtgccgcc tggtcaagta cctgcaggtg     360
   gtgggcatgt tcgcctccac ctacctgctg ctgctcatgt ccctggaccg ctgcctggcc     420
   atctgccagc cgctgagggc gctgcgccgc cgcgcggacc gcctggccgt gctcgccacg     480
   tggctgggct gcctggtggc cagcgcgccg caggtgcaca tcttctcgct gcgcgaggtg     540
   gccgacggcg tcttcgactg ctgggccgtc ttcatccagc cctgggggcc caaggcctac     600
   gtcacgtgga tcacgctctc cgtctacatc gtgcccgtca tcgtgctcgc cgcctgctac     660
   ggcctcatca gcttcaaaat ttggcagaac ctgcgactca agacggcggc ggcggcggcc     720
   gcggaggggc gggagggcgc ggggcgcgcg gccctggctc gggtcagcag cgtcaagctc     780
   atctccaagg ccaagatccg caccgtgaag atgaccttca taatcgtgct ggccttcatc     840
   gtgtgctgga cgccgttctt cttcgtgcag atgtggagcg tctgggacgc cgatgcgccc     900
   aaggaagcct cggctttcat catagccatg ctcctggcca gcctcaacag ctgctgtaac     960
   ccttggatct acatgctctt cacgggccac ctcttccatg aacttgtaca gcgcttcctc    1020
   tgctgctcct ccagctacct gaaggggaac cgtccagggg agaccagcgt cagcaaaaag    1080
   agcaactcat ccacctttgt cctgagccac cacagctcca gccagaggag ctcctcgcag    1140
   ccatccacag tgtga                                                     1155
<210> 15
   <211> 1833
   <212> DNA
   <213> Bubalus bubalis
<400> 15
   atgcaggtcc ccagccccag cgtgcgcgag gcggcctcca tgtacggcac cgcggtggcc 60
   gtcttcctgg tcatcctcgt ggctgcgctg cagggctcgg cccccgccga gagccccttc 120
   cccttccaca tccccctgga ccccgagggg accctggagc tgtcctggaa cgtcagctac 180
   gcgcaggaga ccgtctactt ccagctcctg gtgcgggagc tcaaggctgg tgtcctgttc 240
   gggatgtcgg accgagggga gctggagaat gctgatctgg tggtgctctg gactgacagg 300
   gacggcgcct acttcgggga tgcctggagt gaccagaagg ggcaggtcca cctggactcc 360
   cagcaggatt accagcttct gcgggcacag aggactccag aaggcctgta cctgctcttc 420
   aagaggcctt ttggcacctg tgaccccaac gactacctca tcgaggacgg caccgtccac 480
   ctggtgtacg gactcctgga ggagccgctc cggtcgctgg aggccatcaa catgtccggc 540
   ttgcgcacgg ggctgcagag ggtgcagctg ctgaagccca gcatcctgca gccggccctg 600
   cccacagaca cacgcaccat ggagatccgc gcccctgacg tcctcatccc tggccagcag 660
   accacgtact ggtgctacgt gaccgagctc ccagacggct tcccccggca ccacatcgtc 720
   atgtacgagc ccatcgtcac cgagggcaac gaggcgctgg tgcaccacat ggaggtcttc 780
   cagtgcgccg ccgagttcga gaccatcccc cacttcagcg ggccctgcga ctccaagatg 840
   aagccgcagc ggctcaactt ctgccgacac gtgctggccg cctgggccct gggcgccaag 900
   gccttttact acccagagga agcgggcctg gccttcgggg ggcccggctc ctccagattt 960
   ctccgcctgg aagttcacta ccacaacccg ctggtgataa caggccggcg cgactcctcg 1020
   ggcatccgcc tgtactacac ggccgcgctg cggcgcttcg acgcgggcat catggagctg 1080
   ggcctggcgt acacgcctgt gatggccatc cccccgcagg agacggcctt cgtcctcacc 1140
   ggctactgca cagacaagtg cacccagctg gccctgcccg cctcggggat tcacatcttc 1200
   gcctctcagc tccacacgca cctgacgggc cggaaggtgg tcacggtgct ggccagggac 1260
   ggccgggaga cagagattgt gaacagggac aaccattaca gcccgcactt ccaggagatc 1320
   cgcatgttga agaaggtcgt gtctgtccac ccgggagaca tgctcatcac ctcttgcacg 1380
   tacaacacgg aagacaggag gctggccacc gtggggggct tcgggatcct ggaggagatg 1440
   tgcgtcaact acgtgcacta ctacccccag acgcagctgg agctctgcaa gagcgccgtg 1500
   gaccctggct tcctgcagaa gtacttccac cttgtgaaca ggttcaacag tgaggaagtc 1560
   tgcacctgcc cccaggcgtc tgtccccgag cagtttgcct ccgtgccctg gaactccttc 1620
   aaccgcgagg tgctcaaggc cctgtacggc ttcgcgccca tctccatgca ctgcaacaag 1680
   tcctcggccg tccgcttcca gggcgagtgg aatcggcagc ccctgcctga gatcgtgtcc 1740
   aggttggaag agcccacccc tcactgcccg gccagccagg ctcagagccc cgctggcccc 1800
   accgtgctca acatcggtgg gggcaaaggt tga 1833
<210> 16
   <211> 1352
   <212> DNA
   <213> Equus caballus
<400> 16
   cgccgcggac gcggacgggc tgctggcggg gcgcgggccg ggtgcgggcg gcggtgcggg 60
   gacccctggg gcggcggcgg cgttggcggg gggcgtgctg ctcatcggcg cagtgctcgc 120
   ggggaactcg ctcgtgtgcg tgagcgtggc ggccgagcgc tccctgcaga cgcccaccaa 180
   ctactttatc gtgagcctgg cggccgccga ccccctactc gccctgctcg tgctgcctct 240
   cttcgtctac tccgaggtcc agggcggcgt gtggctgctg agccccggcc tctgcgacgc 300
   gctcatggcc atggacgtca tgctgtgcac cgcctccatc ttcaacctgt gcgccatcag 360
   cgtggacagg ttcgtggccg tggccgtgcc cttgagctac aaccgccaga gccggggcgg 420
   ccgtcagctg ctgctcatcg gcgccacgtg gctgctgtcg gcggcggtgg ccgcgcccgt 480
   gctgtgcggc ctcaacgacg ctcacggccg cgaccccgcc gtgtgccgcc tggaggaccg 540
   cgactacgtg gtctactcat ccgtgtgctc cttcttcctg ccctgcccgc tcatgctgct 600
   gctctactgg gccaccttcc ggggcctgcg gcgctgggag gccgcccgcc gcgccaagct 660
   gcacggccgt gccccgcacc ggaccagcgg cccgggccct ccggcccccg acgccaccgc 720
   gctccggggc ccgctgcccc ccgacgccat cccgccccct gggccacagc ccccagacac 780
   aacccagctc cccagggcca tcacgtctcc caacgccatc ccgttctccg acgccaatgc 840
   gcacccggac gcaacccagc tccccagtgt caccccggcc tccgacgcca ccccgccccc 900
   cgacgccacc gcaccccctg acgccacccc gccccctgac gccatcctgt accccgacgc 960
   cactgcaccc cctgacgcca ccctgccccc cgacgccatc ctgtccccgg atgccatccc 1020
   gtcccccgat gccatcccgt gccccgatgc cgtcgcaccc ctccacgcca tcccggccga 1080
   gcccccgctg caggcccgca ggaggaggcg cgccaagatc accggccggg agcgcaaggc 1140
   catgggggtc ctgcctgtgg tggtcggggt cttcctgctg tgctggtcgc ccttcttcgt 1200
   cgtgcacatc acgcgggcac tgtgtcccac ctgcgcggtg tccccgcggc tggtcagcgc 1260
   agtcacctgg ctgggctacg tcaacagcgc cctcaacccc gtcatctaca ccgtcttcaa 1320
   cgccgagttc cgcaacgtct tccgcaaggc cc 1352
<210> 17
   <211> 522
   <212> DNA
   <213> Eagle
<400> 17
   atggatatca ccattcataa ccccctgatc cgcagacccc tgttatcttg gttggcacca        60
   agtcgtatct ttgaccagat ttttggagag cacctgcagg agtcagagct gctccctgct       120
   tcccccagcc tcagtccctt cctgatgaga tcccccattc ttcggatgcc cagttggcta       180
   gagacaggac tctctgagat gcgactggag aaggacaaat tttctgtaaa tcttgatgtg       240
   aagcatttct cccctgagga gctaaaagtg aaggtgcttg gggacatgat agaaattcat       300
   gggaaacacg aggagcgcca ggatgagcat ggctttattg ccagggagtt caacaggaaa       360
   tacagaattc cagatgatgt ggaccctctg accataacct catcgctctc tctggatggt       420
   gtcctgactg tgagtgcacc aaggaaacaa agtgatgtcc ccgagcgctc tattcctatc       480
   acccgtgaag agaagcctgc cattgcagga gcccaaagga ag                           522
<210> 18
   <211> 390
   <212> DNA
   <213> Bamboo
<400> 18
   atggcgaaga agattgtgca agcggccaac ttgaagcaga tcctcaggag gtgctcgagc        60
   ctggggagga ggcagcagga cttggtgccc aagggccact tcccggtcta cgtcggcgag       120
   agccggtgcc ggttcgtcgt gccgatcgcc tgcctggagc acccggagtt cctggtgctg       180
   ctgcggaagg ccgaggagga gttcgggttc gagcacgacg ccgccatcac gctgccctgc       240
   aatgaggccg tcttcgaggc tgtcctcgcc tccttgacgg gtccaagctc ttccttcttc       300
   cctgatctta gagaccggct ctatagacga ggagaatcga cgtttgaaga tcctggtatt       360
   gcgctctatc cagatatccc aagtgattag                                       390

## Description

This invention relates to formulations comprising oligonucleotides, in particular to food, cosmetic and medicinal formulations. The used oligonucleotides originate from wildlife animals but are obtained via molecular biological means; thus, the invention will help to conserve these animals. In one aspect of the invention, oligonucleotides are added in the form of DNA carrier complexes. Another aspect of the invention is directed at methods of producing the food, cosmetic and medicinal formulations comprising the aforementioned oligonucleotides.

Many components used in traditional Chinese medicine, which knows over 6000 different ingredients, are taken directly from natural occurring sources, be it minerals, animals, plants or herbs. These extracts can cure diseases, promote longevity and improve general health and well-being. Thanks to a longstanding medical tradition the effect of these ingredients has been well proven and documented. However, following industrialization and the spread of traditional Chinese medicine all over the world, the extensive use of natural resources has brought some of the species from which the extracts are taken to the brink of extinction.

In recent years, biologists, chemists and other researches have directed their efforts to deciphering and annotating the genomes of a multitude of animal and plant species (e.g. for the tiger genome, see Cho et al., 2013, Nature Communications, vol. 4, article number 2433, pp. 1-7). This has led to the discovery of many novel gene sequences, among which are also many genes found in animals and plants that are used in traditional Chinese medicine. With the help of homology analysis in combination with activity assays and cell studies, it is possible to attribute a function to some of these newly discovered genes.

These novel findings and discoveries can then be combined with the knowledge of traditional Chinese medicine to identify a connection between the observed effect and possible candidate genes that can subsequently be isolated or produced *in vitro.* Not only will this help to provide a larger patient group with useful natural ingredients, but at the same time, the present invention will contribute to the protection of endangered wildlife, as it offers a possibility to obtain the necessary ingredients by means other than killing animals.

For example, various parts of the tiger are popular for the preparation of Chinese pharmacopeia because of their purported anti-inflammatory effects as well as their healing properties in connection with ulcer, rheumatic pain and burns. The regenerative organs of the tiger, in particular the penis, are prescribed as an aphrodisiac. Possible candidate genes and genomic regions that will provide a similar effect are the growth hormone 1 (GH1), the sex determining region of the Y chromosome (SRY) and a gene involved in the final steps of testosterone synthesis (HSD17B3) of *Panthera tigris altaica* (tiger), which have been identified as the sequences depicted in SEQ ID NO:1, 2 and 3, respectively.

The horn of the rhinoceros is used to treat fevers, convulsions and gout; in some countries, the horn is also used as an aphrodisiac. The main component of the rhinoceros horn is keratin, which is encoded by structural keratin type II (HIY3) of *Buceros rhinoceros silvestris* (rhinoceros) (Hieronymus TL, Witmer LM, Ridgely RC. (2006) Structure of white rhinoceros (Ceratotherium simum) horn investigated by X-ray computed tomography and histology with implications for growth and external form. J Morphol. 267(10):1172-6.); cf. SEQ ID NO:4.

Shark fins are considered to boost sexual activity, promote energy, lower cholesterol levels and rejuvenate the body. The collagen type XIII alpha1 structural protein of *Callorhynchus milii* (shark) has been identified as depicted in SEQ ID NO:5. Collagen is the main component of shark fins (Motta PJ. (1977) Anatomy and Functional Morphology of Dermal Collagen Fibers in Sharks. Copeia. 3:454-464).

Bamboo extracts are found useful in the treatment of epilepsy, febrile conditions and vomiting. Furthermore, bamboo is known for its rapid growth, a trait that is associated with small auxin up RNA 8 as seen in SEQ ID NO:18 (Peng Z, Zhang C et al. (2013) Transcriptome Sequencing and Analysis of the Fast Growing Shoots of Moso Bamboo (Phyllostachys edulis). PLoS ONE. 8(11):e78944. doi:10.1371/journal.pone.0078944.).

Another aspect of Chinese culture are the 12 animal signs. Each of these zodiacs is associated with specific character traits; the annotation of the genomes of the respective species enables scientists to associate these traits with particular genes.

For example, the rat is known for its ambition; this trait is related to the 3 beta-hydroxysteroid dehydrogenase, which is essential for steroid hormone formation; cf. SEQ ID NO:6 (Lachance Y, Luu-The V et al. (1990) Characterization of human 3 beta-hydroxysteroid dehydrogenase/delta 5-delta 4-isomerase gene and its expression in mammalian cells. J Biol Chem. 265(33):20469-75.).

In sheep, the oxytocin receptor (OXTR), which is related to empathy, has been identified as the sequence of SEQ ID NO:7 (Tost H, Kolachana B et al. (2010) A common allele in the oxytocin receptor gene (OXTR) impacts prosocial temperament and human hypothalamic-limbic structure and function. Proc Natl Acad Sci U S A. 107(31):13936-41.doi: 10.1073/pnas.1003296107.).

In ape, the formin-binding protein I (FNBP1L), which is associated with intelligence, has been identified as the sequence depicted in SEQ ID NO:8 (Benyamin B, Pourcain B et al. (2014) Childhood intelligence is heritable, highly polygenic and associated with FNBP1L. Molecular psychiatry. 19(2):253-258. doi:10.1038/mp.2012.184.).

In pig, oxytocin, which is also related to generosity, has been described as the sequence of SEQ ID NO:9 (Barraza JA, Zak PJ. (2009) Empathy toward strangers triggers oxytocin release and subsequent generosity. Ann N Y Acad Sci. 1167:182-9. doi: 10.1111/j.1749-6632.2009.04504.x).

The rooster is valued for executive functioning, which is related to the gene WDR72; cf. SEQ ID NO:10 (LeBlanc M, Kulle B et al. (2012) Genome-wide study identifies PTPRO and WDR72 and FOXQ1-SUMO1P1 interaction associated with neurocognitive function. J Psychiatr Res. 46(2):271-8. doi: 10.1016/j.jpsychires.2011.11.001.).

The dragon is known for intelligence; in its closest living relative, the *Varanus comodovarensis,* brain plasticity can be traced back to the gene BDNF as seen in SEQ ID NO:11 (Binder DK, Scharfman HE. (2004) Brain-derived Neurotrophic Factor. Growth factors (Chur, Switzerland). 22(3):123-131. doi:10.1080/08977190410001723308.).

The Chinese zodiac rabbit possesses creativity, which is based on neuregulin 1 as seen in SEQ ID NO:12 (Kéri S. (2009) Genes for psychosis and creativity: a promoter polymorphism of the neuregulin 1 gene is related to creativity in people with high intellectual achievement. Psychol Sci. 20(9):1070-3. doi: 10.1111/j.1467-9280.2009.02398.x.)

The snake is known to strike quickly, a trait that is related to the gene encoding acetylcholinesterase (AChE). AChE promotes nerve functioning and development and is encoded in the snake by SEQ ID NO:13 (Sternfeld M, Ming G et al. (1998) Acetylcholinesterase enhances neurite growth and synapse development through alternative contributions of its hydrolytic capacity, core protein, and variable C termini. J Neurosci. 18(4):1240-9.).

The dog is cherished for loyality, a trait that is related to oxycytocin (Scheele, D. et al. (2012) Oxytocin Modulates Social Distance between Males and Females. J. Neurosci. 32: 16074-16079.). The sequence of the corresponding oxytocin receptor is shown in SEQ ID NO:14.

The buffalo's trait of perseverance and the ability to focus on one task is related to the formation of 2-phenylethylamine, which is controlled by dopamine beta-hydroxlase (Irsfeld M, Spadafore M, Prüß BM. (2013) β-phenylethylamine, a small molecule with a large impact. WebmedCentral. 4(9):4409.); cf. SEQ ID NO:15.

The horse is known to seek novelty and to be curious; this is connected to the dopamine receptor D4 (Munafò, M. R. et al. (2008) Association of the Dopamine D4 Receptor (DRD4) Gene and Approach-Related Personality Traits: Meta-Analysis and New Data. Biological Psychiatry 63:197-206), which can be seen in SEQ ID NO:16.

Another animal known for its outstanding qualities in particular with regard to eye sight is the eagle. Here, the relevant gene is crystallin beta, which is a component of the eye lense and is depicted in SEQ ID NO:17.

While all of the above mentioned sequences have been deciphered and annotated, their effect on the human body has not been elucidated so far. In fact, prior to the invention, introduction of particular gene sequences from any origin other than human into humans has not been considered. Thus, it is an objective of the present invention to provide active ingredients of animal and plant extracts in the form of isolated mammalian, reptilian, avian and plant, non-human gene sequences for the administration to humans. By providing the active ingredients of known traditional formulations in the form of *in vitro* produced oligonucleotides, killing of animals is circumvented and animal wildlife thus protected.

Furthermore, using whole animals or parts thereof poses a risk since it involves uptake of the complete animal or plant genome, which might harbor viral sequences. Additionally, the extract might be contaminated with pathogens. It has been shown that such viruses and pathogens are involved in the development of cancers (Burnett-Hartman AN, Newcomb PA et al. (2008) Infectious Agents and Colorectal Cancer: A Review of Helicobacter pylori, Streptococcus bovis, JC Virus, and Human Papillomavirus. Cancer Epidemiol Biomarkers Prev. 17: 2970-2979. doi: 10.1158/1055-9965.EPI-08-0571). Thus, providing defined, synthetic, virus-free oligonucleotides ensures a high level of medical safety and can protect individuals from harmful agents.

Previously, DNA spheres containing DNA probes of around 100 nucleotides have been described (US 8,835,178 B2). These probes are used in scientific setups as test reagent for atmospheric release and bio-hazard detection; in contrast to the present invention, the DNA within the spheres is not expressed, but merely detected via PCR.

Other systems have been coined for the delivery and expression of nucleic acid molecules (US 7,030,097 B1). This system consists of a polymeric structure formed by a biocompatible polymer and a mixture of nucleic acid molecules and a co-dispersant. Using this system, DNA can be delivered into an organism, but only with the help of additional polymers, thus complicating the production process and additionally introducing foreign structures that might elicit immune responses.

Until recently, it was believed that delivery of "naked" DNA, *i.e.* DNA without any uptake promoting agents, does not result in efficient levels of DNA expression (Villemejane J, Mir LM. (2009) Physical methods of nucleic acid transfer. Brit J Pharma. 157: 207-219.). However, it has now been shown that complete genes may be taken up via the digestive tract (Spisák S, Solymosi N et al. (2013) Complete Genes May Pass from Food to Human Blood. PLoS ONE 8(7): e69805. doi: 10.1371/journal.pone.0069805). Additionally it has been shown that the bacterial communities inhabiting the human gut can take up DNA and express these genes as functional products (Hehemann, JH et al. (2010) Transfer of carbohydrate-active enzymes from marine bacteria to Japanese gut microbiota. Nature 464: 908-91. doi: 10.1038/nature08937). Moreover, formulations comprising DNA and aiming to achieve immune effects in humans are known in the art. For example, Roy et al. (1999, Nature Medicine, vol. 5(4), pp.387-391) disclose formulations with stabilised DNA of peanut allergen gene for oral gene delivery and immunisation. Oliveira et al. (2012, The Scientific Journal, vol. 164(12), pp. 6406-6411) disclose oral vaccination based on DNA-chitosan nanoparticles against Schistosoma mansoni infection.

Accordingly, it is an object of the present invention to provide the respective genes from animal sources in a formulation that allows for expression of these genes and not just delivery in a human individual, while at the same time refraining from using any superfluous carrier polymers.

The object is solved by a formulation comprising at least 1 million oligonucleotides of mammalian, non-human DNA, which oligonucleotides have at least 80% homology to one or more of the sequences selected from the group consisting of SEQ ID NOs: 1 to 3.

Throughout the specification, the term "oligonucleotide" is to be conceived as comprising single-stranded or double-stranded oligonucleotides or a mix thereof. The oligonucleotides may also consist of RNA. Each oligonucleotide may have a length of 10 to 2500 bp and may also be a concatemer of the same oligonucleotide in repetition.

In the present specification, the terms "homology" and "homologous" are meant to be understood as sequence identity. Homology between amino acid sequences or nucleotide sequences can be examined using the algorithm BLAST (Altschul, SF, Gish W et al. Basic local alignment search tool. (1990) J. Mol. Biol. 215:403-410.) and its further developed versions BLASTN and BLASTX. Default parameters should be employed for all three programs.

Throughout this specification and the claims which follow, unless the context requires otherwise, the word "comprise" and variations such as "comprises" and "comprising" will be understood to imply in inclusion of a stated integer or step or group of integers but not the exclusion of any other integer or step or group of integers or steps. As used in this specification and the appended claims, the singular forms "a", "an" and "the" include plural referents, unless the context clearly dictates otherwise.

Surprisingly it was found that the use of 1 million oligonucleotides is more efficient than the uptake of the related extract in terms of promoting the expression of the relevant gene. This is most likely due to the high copy number of the respective genes in comparison with only small amounts of the respective gene sequences that are present in traditionally prepared extracts.

Within the scope of the invention, it is envisaged that the formulation may comprise 1, 1.5, 2, 2.5, 3, 3.5, 4, 4.5, 5, 6, 7, 8, 9 or 10 million oligonucleotides. Preferably, the formulation of the invention comprises 1, 2 or 3 million oligonucleotides; most preferably, the formulation of the invention comprises 1 million oligonucleotides.

The oligonucleotides can have a sequence homologous to one or more of the sequences selected from the group consisting of SEQ ID NOs: 1 to 3, so that different types of oligonucleotides are present in the formulation. For example, the one or more sequences of the oligonucleotide can be at least two, at least three, at least four or at least five different sequences. In embodiments where two or more different types of oligonucleotides are present in the formulation, the formulation comprises 1 million copies of each of the different oligonucleotides. It is envisaged that in embodiments comprising oligonucleotides with two or more different sequences, the different sequences are from genes likely to show a similar effect; e.g., in one embodiment, oligonucleotides with SEQ ID NOs: 1, 2 and 3 are combined. In embodiments where genes with a similar effect are combined the overall effect on the individual will be even greater than if each type of oligonucleotide is taken separately.

Oligonucleotides used in the formulation of the present invention can have 80% or more homology to SEQ ID NOs: 1 to 3. In a preferred embodiment, the oligonucleotides have more than 90% homology to SEQ ID NOs: 1 to 3. In the most preferred embodiment, the oligonucleotides have more than 95% homology to SEQ ID NOs: 1 to 3. It is further envisaged that the oligonucleotides might contain "silent" mutations. A "silent mutation" is hereby defined as a change of the nucleotide sequence that, due to the ambiguity of the genetic code, does not result in a change of the amino acid sequence upon expression. Silent mutations may be introduced because of differences in codon usage between different species, *i.e.* frequency of the occurrence of synonymous base triplets all encoding for the same amino acid.

To practice the present invention, unless otherwise indicated, conventional methods of chemistry, biochemistry, cell biology and recombinant DNA techniques are employed which are explained in the literature in the field (cf., e.g., Molecular Cloning: A Laboratory Manual, 4th Edition, Green and Sambrook eds., Cold Spring Harbor Laboratory Press, Cold Spring Harbor 2012).

The oligonucleotides of the invention may be obtained by all methods generally applied in the field, e.g., polymerase chain reaction using genomic DNA or plasmid DNA as a template, reproduction in bacteria or cells or *in silico* synthesis. When oligonucleotides are expressed in recombinant systems, the skilled person is capable of designing a suitable system including an appropriate promoter and plasmid vector as well as a cell line or bacterial strain that can express the oligonucleotide used in the formulation of the invention. For example, the plasmid constructs used to this extent may comprise a suitable promoter with high activity in the respective cell line or bacterial strain and further transcription and translation regulators as well as suitable linkers with appropriate restriction sites. The skilled person is also aware of the influence of culture conditions on plasmid yield.

If necessary, produced oligonucleotides can be purified by precipitation, with the help of commercially available purification kits (e.g. Wizard® PCR Preps DNA Purification System, Promega, Madison, WI, USA) or via agarose gel electrophoresis. The person skilled in the art is well aware of how each of these methods can be optimized to maximize oligonucleotide yield and purity.

In order to ensure long-term integrity and stability of the oligonucleotides within the formulation, additional agents inhibiting the degradation of the oligonucleotides can be added to the formulation. These include DNase inhibitors in the form of specific proteins, nucleotides, anthracycline and aminoglycoside antibiotics, synthetic organic and inorganic compounds or commercially available DNase removing agents (Ambion® DNase Removal Reagents, Life Technologies, Carlsbad, CA, USA).

The oligonucleotides may also be modified themselves to increase their stability. Possible modifications include phosphorothiorate, methylphosphonate, phosphoroamidate, boranophosphate, phosphoroacetate or 2',5'-phosphodiester. Amide-linked oligonucleotides, morpholino and peptide nucleic acids are also contemplated in the scope of the invention.

Furthermore, the formulation of the invention may also comprise cellular uptake agents. Cellular uptake agents are molecules, ligands or moieties that facilitate uptake of the oligonucleotide into cells. The cellular uptake agents can be independent of the oligonucleotides or fused to one or more of them. One group of cellular uptake agents includes lipids and cationic lipids. Another group of cellular uptake agents are receptor binding ligands that can recognize surface molecules on cells. In general, all molecules that target markers expressed on the surface are contemplated as cellular uptake agents; in particular, growth factors, cytokines and antibodies may be used. Fragments of suitable ligands may also be used in the formulations and methods of the present invention.

The cellular uptake agents can be covalently or non-covalently linked to the oligonucleotides. Covalent linkage can be based on UV crosslinking or chemical crosslinking, whereas non-covalent attachment can be achieved by exploiting electrostatic or hydrophobic interactions. All of these methods have been previously described and are well known to the skilled artisan.

The formulation of the invention can be administered alone or in combination with a physiologically acceptable carrier, e.g. solvents, perfumes or colorants, sunscreens or other ingredients for medicinal or cosmetic use. Furthermore, vehicles such as water, saline or other delivery vehicles can be added to the formulation. It is also envisaged that the formulation of the present invention is mixed with commercially available "energy" drinks such as RedBull®, Monster Energy®, Rockstar®, NOS® or Amp Energy®.

In another aspect of the invention, the formulation contains the oligonucleotides in the form of DNA carrier complexes comprising oligonucleotides and a physiologically acceptable stabilizer. This results in the oligonucleotides being protected from nuclease activity after application or administration and thus, in an increase of the number of intact oligonucleotides that can be expressed within the cells of the individual. Because a higher percentage of the oligonucleotides remain intact for a longer time, the use of a DNA carrier complex makes it possible to reduce the overall copy number of oligonucleotides.

The person skilled in the art is well aware how such DNA carrier complexes can be formed and how to choose a suitable stabilizer material. Physically acceptable stabilizer include sodium or potassium alginate, hyaluronic acid, poly(ethylene-co-vinyl acetate), poly-L-lactide, poly-D-lactide, polyglycolide, poly(lactide-co-glycolide), polyanhydride, polyorthoester, polyphosphazene, proteinaceous polymer, polyester, silicone, or combinations thereof.

Depending on the mode of application, the DNA carrier complexes can be appropriately sized in the range between 5 and 500 nm.

The DNA carrier complexes may also comprise cellular uptake agents, which can be covalently linked or non-covalently attached to the DNA carrier complex.

In another embodiment, the present invention relates to a cosmetic formulation or a topical dermatological formulation. The cosmetic formulation of the present invention allows for topical application of the oligonucleotides, resulting in local expression thereof. This leads to an increased effect in particular regions of the body, whereas no side effects are observed in other body parts.

Cosmetic formulations according to the present invention may comprise preservatives such as esters of p-hydroxybenzoic acid, e.g. methyl- p-hydroxybenzoate, and propyl p-hydroxybenzoate; cis-1-(3-chloroallyl)-3,5,7-triaza-1-azoniaadamantane chloride; ethylenediaminetetraacetic acid (EDTA) and salts of EDTA; imidazolidinyl urea; sodium N-lauryl-p-iminodipropionate and the like or any combination thereof. Additionally, color and essence can be added to the cosmetic formulation of the present invention. Color additives include both natural and artificial dyes, e.g., carotenoid derivatives and iron oxides, while essences can include any non-irritating natural and artificial oils and perfumes. In general, the individual ingredients used in the formulation should be of a quality or purity suitable for cosmetic use and the cosmetic formulation will be non-stinging and non-irritant.

In yet another embodiment, the present invention relates to a food formulation and a beverage food formulation. Food and beverage food formulations of the present invention enable the convenient uptake of a large amount of the respective oligonucleotides and are particularly useful when the desired effect is not limited to specific body parts. They may additionally comprise natural and artificial sweeteners such as sugar, mannitol, sorbitol, xylitol, lactitol, isomalt, maltitol and hydrogenated starch hydrolysates (HSH); salt; flavor enhancers; acidifiers; electrolytes; and emulgators. They may also comprise whole grains, nuts, protein sources, seeds, dried fruits or other nutrients.

Furthermore, it is envisaged that the beverage food formulation of the present invention comprises stimulating agents such as caffeine, guarana or taurine and additional ingredients such as herbal extracts from, e.g., green tea, ginseng and gingko biloba, as well as vitamins such as B vitamins and sugar. Combination of the beverage food formulation of the present invention taken with stimulating agents further enhances the effect of the oligonucleotides, in particular where this effect is connected to male potency.

The beverage food formulation of the invention may be premixed with the oligonucleotides or the oligonucleotides may be contained in the beverage food formulation as a separate entity or may be provided to the customer in a form that allows for ad hoc mixture of the beverage food formulation. If the oligonucleotides are present as a separate entity, they may be contained in a capsule, a gelous ball (e.g. comprising tapioca) or a pill within the beverage food formulation. If the oligonucleotides are provided in a form allowing for ad hoc mixture by the customer himself, they may be, for example, provided within a sachet, a teabag, an injector or a dispenser that is provided together with a conventional beverage container such as a can or a bottle. It is also envisaged that the injector or dispenser is directly connected to the food beverage container.

In one embodiment, the present invention relates to a medicinal formulation. The medicinal formulation of the present invention may additionally comprise a suitable pharmaceutical excipient such as starch, glucose, lactose, sucrose, gelatin, malt, rice, flour, chalk, silica gel, sodium stereate, glycerol monostereate, talc, sodium chloride, dried skim milk, glycerol, propylene, glycol, water, ethanol and the like.

In further embodiments, the present invention relates to formulations, wherein the sequence of the oligonucleotides is SEQ ID NO:1, 2, or 3.

In one embodiment of the invention the oligonucleotides have the sequence of SEQ ID NO:1. Application or administration of this formulation leads to increased potency, stimulates libido and improves sexual performance, particularly in male individuals.

In another embodiment of the invention the oligonucleotides have the sequence of SEQ ID NO:2. Application or administration of this formulation leads to increased potency, stimulates libido and improves sexual performance, particularly in male individuals.

In yet another embodiment of the invention, the oligonucleotides have the sequence of SEQ ID NO:3. Application or administration of this formulation leads to increased potency, stimulates libido and improves sexual performance, particularly in male individuals.

Reference oligonucleotides have the sequence of SEQ ID NO:4. Application or administration of this formulation helps with febrile conditions and convulsions and also stimulates sexual activity.

Other reference oligonucleotides have the sequence of SEQ ID NO:5. Application or administration of this formulation improves the sexual appetite, boosts energy levels and helps to rejuvenate the body.

Still other reference oligonucleotides have the sequence of SEQ ID NO:6. Application or administration of this formulation leads to increased steroid hormone expression, resulting in increased ambition in the individual.

Still other reference oligonucleotides have the sequence of SEQ ID NO:7. Application or administration of this formulation leads to higher levels of the oxytocin receptor, which impacts social behavior.

Still other reference oligonucleotides have the sequence of SEQ ID NO:8. Application or administration of this formulation leads to higher expression levels of formin-binding protein 1-like protein and thus modulates brain development and activity.

Still other reference oligonucleotides have the sequence of SEQ ID NO:9. Application or administration of this formulation leads to increased production of oxytocin, which influences social bonding and interaction.

Still other reference oligonucleotides have the sequence of SEQ ID NO:10. Application or administration of this formulation leads to higher expression levels of WDR72, which promotes neurocognitive functioning.

Still other reference oligonucleotides have the sequence of SEQ ID NO:11. Application or administration of this formulation leads to increased expression of brain-derived neutrophic factor and, thus, enhances brain plasticity.

Still other reference oligonucleotides have the sequence of SEQ ID NO:12. Application or administration of this formulation leads to increased levels of neuregulin 1, promoting creativity.

Still other reference oligonucleotides have the sequence of SEQ ID NO:13. Application or administration of this formulation results in overexpression of AChE, which positively influences nerve functioning and development.

Still other reference oligonucleotides have the sequence of SEQ ID NO:14. Application or administration of this formulation results in expression of OXTR, which will increase empathy in individuals.

Still other reference oligonucleotides have the sequence of SEQ ID NO:15. Application or administration of this formulation increases expression of dopamine beta-hydroxlase and therefore improves power of concentration.

Still other reference oligonucleotides have the sequence of SEQ ID NO:16. Application or administration of this formulation leads to enhanced expression of the dopamine receptor D4, making individuals more likely to take risks.

Still other reference oligonucleotides have the sequence of SEQ ID NO:17. Application or administration of this formulation leads to higher levels of crystalline beta, thus improving eye-sight.

Still other reference oligonucleotides have the sequence of SEQ ID NO:18. Application or administration of this formulation will promote growth.

Furthermore, it is envisaged to use the formulation of the invention in one embodiment in personal lubricants. These lubricants can be water-based, oil-based or silicone-based. A sufficient amount of the formulation of the invention is added so that 1 ml contains at least 1 million oligonucleotides. The personal lubricant in which the formulation of the invention is used may contain any of the ingredients compatible with a personal lubricant such as buffer salts, clouding agents, anticaking agents, flavors, inorganic base, water-soluble cellulose-derived polymers, polyhydric alcohol and color.

The personal lubricant comprising the formulation of the invention may be prepared conventionally or be adapted to specific needs at the manufacturing site. Conventional preparation consist of mixing all the required ingredients by dissolving them in water of appropriate purity and adding the rehydrated oligonucleotide in varying concentrations for example from 1 million to 10 million oligonucleotide molecules per ml of personal lubricant, depending on the desired strength. The oligonucleotide formulation of the invention may be added by dripping the rehydrated oligonucleotide solution into the lubricant solution, followed by a mixing step.

In yet another embodiment, the present invention relates to a method to produce the formulations of the present invention.

Lyophilized oligonucleotides of SEQ ID NOs:1-3 can be rehydrated with an appropriate aqueous buffer system such as monopotassium phosphate (E340) or potassium tartrate (E336) buffer adjusted to pH 8, in order to reduce depurination of the oligonucleotide. The resulting solution can be added to yield the formulations of the invention.

As a general means of preparation suitable for most applications, rehydrated oligonucleotides can simply be added as a solution to a certain final concentration, which is dependent on the desired amount of molecules. The amount of molecules is determined as follows: oligonucleotides are quantified by spectroscopic means (e.g. NanoDrop, ThermoFischer, USA) at a wavelength of 260 nm. The result, an amount expressed in nanogram/µl is converted via the following formula: oligonucleotide concentration (ng/µl) / [0.66*DNA size (bp)] for double stranded oligonucleotides and oligonucleotide concentration(ng/µl) / [0.33*DNA size (bp)] for single stranded oligonucleotides, respectively.

Lyophilized oligonucleotides may be mixed with dry or liquid flavouring and/or colouring agents in and may be contained in a container.

The invention also relates to a kit for individual preparation of the formulation of the invention by the end consumer. This kit comprises containers with different sets of lyophilized oligonucleotides, an appropriate buffer system, a measuring device and instructions on how to prepare different strength of the final product and mixing formulations with foods, beverages, medicinal formulation, cosmetic formulation or a topical dermatological formulation.

In another formulation the formulation of the invention can be used in a spice mixture.

It is to be understood that the present invention is not limited to the particular methodology, protocols and reagents described herein, as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meaning as commonly understood by one of ordinary skill in the art.

The invention is furthermore explained by the following figures, which are, however, not intended to limit the scope of the invention.

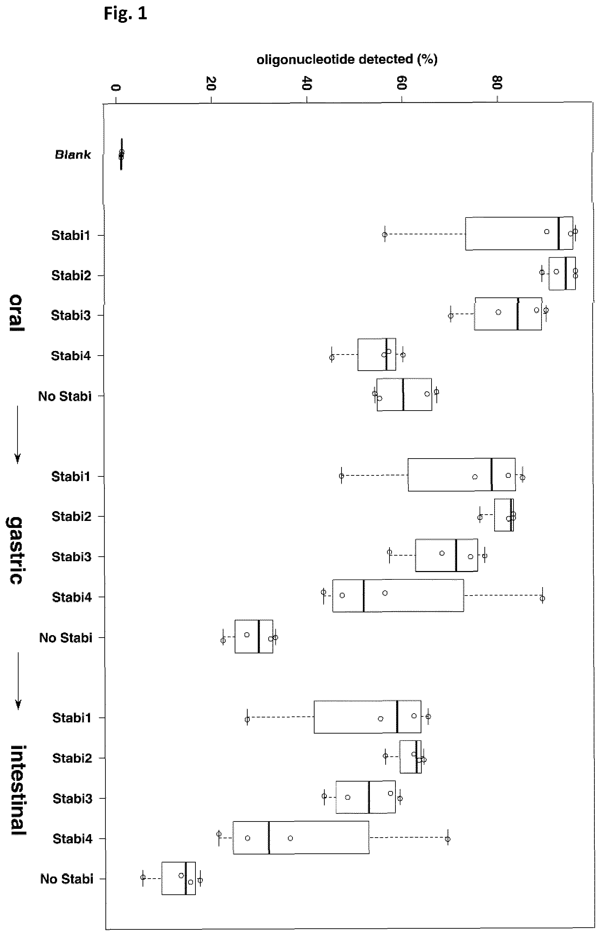

Figure 1 shows the effect of different stabilizers on digestion of the oligonucleotides of the invention during various phases of *in vitro* digestion. Blank: control with buffer only.

The following examples are intended to illustrate the invention.

### Example 1

### Obtaining DNA sequences through DNA sequencing

DNA is extracted from body-fluids or tissues of the animal or plant of interest using commercially available kits such as *Quick*-DNA™ Universal Kit (Zymo Research Europe GmbH, Freiburg, Germany). Shotgun libraries for sequencing are generated using the Nextera XT DNA Sample preparation kit (Illumina, San Diego, USA) following the manufacturer instructions. The whole genome of the corresponding animal or plant is sequenced with the Genome Analyzer Ilx (Illumina, San Diego, USA). The libraries are sequenced in a 112 bp paired end single indexed run. The resulting paired end reads are assembled using SPAdes assembler (Bankevich A et al. (2012) SPAdes: A New Genome Assembly Algorithm and Its Applications to Single-Cell Sequencing, J Comp Biol. 5: 455-77, doi:10.1089/cmb.2012.0021) or MIRA (Chevreux B, Wetter T et al. Genome Sequence Assembly Using Trace Signals and Additional Sequence Information. in German Conference on Bioinformatics, 1999, 45-56.) on an appropriately equipped GNU/Linux computing cluster. In order to identify homologs of human genes with a known function, the contiguous sequences (contigs) are searched with tblastn (E. Michael Gertz et al. Composition-Based Statistics and Translated Nucleotide Searches: Improving the TBLASTN Module of BLAST. BMC Biol. 2006, December 7; no. 1: 41, doi:10.1186/1741-7007-4-41.) using the human protein sequence.

### Example 2

### Production of specific oligonucleotide molecules

The oligonucleotides comprising the sequence of interest (SOI), i.e., SEQ ID NOs: 1 to 18, can be produced by several means:

### 1. Polymerase chain reaction using a thermal cycler

Polymerase chain reaction (PCR) is performed according to the method described previously (Saiki RK et al. Enzymatic Amplification of Beta-Globin Genomic Sequences and Restriction Site Analysis for Diagnosis of Sickle Cell Anemia. Science 1985, December 20. 230, no. 4732: 1350-54, doi:10.1126/science.2999980.). For the amplification of each sequence of interest (SOI) oligonucleotide primers with a length between 20 and 25 bases and always ending in a guanine or cytosine base are chemically synthesized and 100 pmol of each primer are added to 1 µg of genomic DNA template or a plasmid containing a chemically synthesized DNA (Genscript,USA) containing the SOI. 35 amplification cycles are performed in a thermocylcer (DNA Engine Tetrad2, Bio-Rad, USA) using the polymerase chain reaction (PCR) according to the instructions of the manufacturers (OptiTaq, RoboKlon, Germany). Denaturation is performed at 98°C for 30 seconds, primer annealing at 55°C for 25 seconds and primer extension at 72°C for 1 min per kilobase of the SOI. An analytic sample (approx. 2 µl) of the resulting product is analyzed on a 1.0 % agarose gel (CAS-Nr. 9012-36-6) with a DNA-size standard (1 kbp DNA-Ladder, Carl-Roth GmbH + Co. KG, Germany). After staining of the agarose gel (Roti-GelStain, Carl-Roth GmbH + Co. KG, Germany), presence of oligonucleotide of the expected size of the SOI is confirmed on a transilluminator (Benchtop UV Transilluminators, UVP, USA). If unspecific bands other than that of the SOI are detected, PCR is repeated and optimized by varying the annealing temperature, adding enhancers such as PCR-grade DMSO (CAS-Nr. 67-68-5) or by varying buffer compositions and concentration of the utilized template DNA. Upon establishing a highly specific protocol that only amplifies the SOI, upscaling is achieved by increasing the amount of oligonucleotides and deoxynucleotides used and parallelizing the process in 96- or 384 well PCR plates (twin.tec PCR plates, Eppendorf, Germany).

### 1.1 Product purification

The resulting PCR product has to be separated from the DNA polymerase, the buffer components and deoxynucleotides. This is achieved by either using commercially available purification kits (e.g. NucleoSpin Gel and PCR Clean-up, MACHEREY-NAGEL, Germany) following the instructions of the supplier or by means of precipitation. For precipitation, the PCR reactions are pooled and sodium acetate (CAS-Nr. 127-09-3) (0.3 M, pH 5.2, final concentration) is added to the oligonucleotide mix followed by 2 volumes of ice cold absolute ethanol (CAS-Nr. 64-17-5) on 1 volume oligonucleotide, followed by an incubation at -80°C for at least two hours. This precipitation is followed by a 30 minute centrifugation at 17000 x g. After removing the supernatant, the oligonucleotide pellet is washed with 70% ethanol, centrifuged for 15 minutes at 17000 x g, air dried for 30 minutes and resuspended with 2.0 M NaCl (7647-14-5) and 50 mM Tris-CI (CAS-Nr. 1185-53-1). pH is adjusted to 7.5 with HCI (CAS-Nr. 7647-01-0).

### 1.2 Storage

The resulting product is mixed with sucrose (CAS-Nr. 57-50-1) to a final concentration of 20mg/µl and filled into brown hydrolytic class 1 Type FIOLAX glass vials (Schott, Germany) which are closed with butyl rubber lyophilisation stoppers (Wheaton, USA). Lyophilisation is performed as follows: vials are frozen to -21°C, drying is started at a shelf temperature of -21 °C and a chamber pressure of 0.35 mbar and the temperature elevated to -10°C during the drying process. Secondary drying is carried out at a shelf temperature of -21°C and chamber pressure of 0.2 mbar. The resulting product is sealed with temper proof caps (West Flip-Off seals, West Pharmaceutical, Germany). Lyophilised DNA can be stored at 25°C/60% relative humidity (6 months), 2-8°C (24 months), and -20°C (66 months) in the dark without significant degradation (van der Heijden I, Beijnen JH et al. (2013) Long Term Stability of Lyophilized Plasmid DNA pDERMATT. Int J Pharm. 10; 453, no. 2: 648-50, doi:10.1016/j.ijpharm.2013.06.010.).

### 2. Replication in microbial cells

Specific oligonucleotide sequences can be produced in large quantities by integrating them into a replicative plasmid, which can be used to transform bacterial cells, such as *Escherichia coli and Bacillus subtilis,* which, while propagating and dividing, will replicate and propagate the plasmids carrying the SOI.

In order to yield economically feasible amounts of oligonucleotide, high copy numbers per cell, high cell densities and efficient downstream processing methods must be achieved.

The SOI can be combined with a replicative plasmid using standard cloning techniques (e.g. SLIC as described previously (Kumpfmüller J, Kabisch J et al. (2013) An Optimized Technique for Rapid Genome Modifications of Bacillus Subtilis. J Microbiol Met. 3: 350-52, doi:10.1016/j.mimet.2013.10.003)) with an origin of replication (ORI) resulting in high-copy numbers in the cells. Examples for such origins are the ColE1 and pUC ORIs (Prather KJ et al. (2013) Industrial Scale Production of Plasmid DNA for Vaccine and Gene Therapy: Plasmid Design, Production, and Purification. Enz Micro Tech. 7: 865-83, doi:10.1016/S0141-0229(03)00205-9.), or origins with so called "runaway-replication", which, after a temperature shift, lose control of replication regulation (Uhlin BE and Nordström KA. (1978) Runaway-Replication Mutant of Plasmid R1drd-19: Temperature-Dependent Loss of Copy Number Control. Mol General Gen MGG. 165, no. 2: 167-79, doi:10.1007/BF00269904; Remaut E, Tsa H. (1983) Improved Plasmid Vectors with a Thermoinducible Expression and Temperature-Regulated Runaway Replication. Gene. no. 1: 103-13.) and can lead to copy numbers of 1000 per cell for the Gram-negative bacterium *Escherichia coli.* For the Gram-positive bacterium *Bacillus subtilis* the RepE ORI (Swinfield TJ et al. (1990) Physical Characterisation of the Replication Region of the Streptococcus Faecalis Plasmid pAM Beta 1. Gene. 87, no. 1: 79-90.) can be applied. The resulting plasmid carrying the SOI has to be transformed into the appropriate host. Transformation, meaning the transfer of plasmid DNA into bacterial cells, can be achieved through electroporation of *B. subtilis* (Kabisch J et al. (2012) Characterization and Optimization of Bacillus Subtilis ATCC 6051 as an Expression Host. J Biotechnol. doi:10.1016/j.jbiotec.2012.06.034.) and *E. coli* (Dower WJ, Miller JF et al. (1988) High Efficiency Transformation of E.coli by High Voltage Electroporation. Nuc Acids Res. 16, no. 13: 6127-45, doi:10.1093/nar/16.13.6127.) following the protocols in the indicated publications.

The cells harboring the plasmids are cultivated in an incubator shaker using a C-source release medium such as Enpresso (BioSilta Ltd., Cambridgeshire, United Kingdom) or in a fed-batch process using a bioreactor. Several optimized processes have been described for the most commonly applied *E.coli* strains (Quaak SGL et al. (2008) GMP Production of pDERMATT for Vaccination against Melanoma in a Phase I Clinical Trial. Euro J Pharma Biopharma. 70, no. 2: 429-38, doi:10.1016/j.ejpb.2008.05.002.). In short, lysogeny-broth (LB) plates, containing an appropriate antibiotic for keeping selection pressure on plasmid carrying cells, are generously inoculated from frozen glycerol stocks of *E. coli* DH5α strains harboring plasmid pUC with SEQ ID NOs: 1-18. These plates are incubated overnight at 30°C and the resulting bacterial lawn is washed off the plates using two times 2.5 ml pre-warmed EnPressoB medium. The resulting suspension is transferred into a 10I stirred tank bioreactor (Labfors 5, Infors AG, Switzerland) and the cells are cultivated with a set pH of 6.7 and in enzyme controlled glucose limited fed-batch (EnPressoB, Biosilta Oy, Finnland). Before entering the transient phase, the correct time point being determined through previous experiments, cells are harvested by centrifugation with 10000 x g and the plasmids harboring the SOI are isolated as described in Example 2.1.

### 2.1 Product purification

In order to administer the product, key impurities such as proteins, endotoxins, lipopolysaccharides, RNA and chromosomal DNA need to be removed in a scalable and robust process.

Most described plasmid purification protocols consist of three steps: cell lysis, primary purification and concentration through precipitation or filtration followed by a secondary purification step applying one or more polishing chromatography columns.

For isolation, cells harboring the plasmids with the SOI are lysed using alkaline lysis (Bimboim HC and Doly J. (1979) A Rapid Alkaline Extraction Procedure for Screening Recombinant Plasmid DNA. Nuc Acids Res. 7, no. 6: 1513-23, doi:10.1093/nar/7.6.1513.) followed by the above described precipitation. In order to remove impurities in a fast and scalable way, Immobilized Metal Affinity Chromatography (IMAC) is applied. It was shown that free purine bases, as found in RNA and damaged DNA bind, to metal charged resins (e.g. iminodoacetic acid with Cu(II)), while intact plasmid DNA is not retained. In order to release the SOI from the plasmid backbone, the isolated plasmid is cleaved using an appropriate restriction endonucleases (RE), e.g. the RE that was used to ligate the SOI with the replicative plasmid backbone. The SOI is then separated from the replicative plasmid backbone and the RE using a Superose 6 (GE Healthcare, USA) size-exclusion chromatography column.

### 2.2 Storage

The resulting oligonucleotide is stored as described above.

### Example 3

### Product application

Different means of application of the product are possible:

### 1. Mixing with beverages

This includes the re-hydration of the lyophilized oligonucleotides on site of the beverage manufacturers and mixing the oligonucleotide with a beverage to a concentration of at least 1 million molecules of oligonucleotides per serving.

### 2. Reversed spherification

1.5 % w/v calcium-lactate hydrate (CAS Number: 41372-22-9) is dissolved in an edible solution (e.g. mango juice, amaretto) and the respective oligonucleotides are added to this solution in varying concentrations. The resulting suspension is dripped into a 0.5% w/v dispersion with a syringe or a JetCutter (geniaLab, Braunschweig, Germany) of sodium-alginate (CAS Number: 9005-38-3) and deionized water and incubated at room temperature for 3 minutes. The resulting oligonucleotide carrier complexes are washed twice with deionized water and stored in the previously used edible solution.

### 3. Mixing with cosmetics products

The lyophilized oligonucleotide is resuspended together with a salt of the ethylenediaminetetraacetic acid (EDTA), a common chelating agent in cosmetics used for viscosity control. EDTA is a well known stabilizing agent for oligonucleotides. This suspension is mixed with the cosmetic product in a concentration of at least 1 million molecules/ml. Additionally, omega-3-oleic acid can be added in order to increase skin penetration of the oligonucleotide (Touitou, E, Godin, B et al. (2002) Oleic acid, a skin penetration enhancer, affects Langerhans cells and corneocytes. J Con Rel. 80: 1-7. doi:10.1016/S0168-3659(02)00004-4.).

### Example 4

### In vitro digestion of oligonucleotides

In order to simulate the effect of digestion and oligonucleotide uptake without human trials and without harming endangered species, *in vitro* gastro-intestinal digestion experiments were performed. These followed the setup as suggested by a leading consortium (Minekus, M. et al. (2014) A standardised static in vitro digestion method suitable for food - an international consensus. Food Funct. 5, 1113-1124. doi: 10.1039/C3F060702J) using an oligonucleotide with SEQ ID NO: 1. In short, oral, gastric and intestinal passage were simulated by emulating their respective chemical and enzymatic compositions.

Samples were incubated, as suggested, in each phase for 2 minutes and the oligonucleotides were immediately isolated using the QIAamp DNA Micro Kit (Qiagen, Germany) and stored at 4°C until quantification.

The integrity of the oligonucleotides after *in vitro* digestion was determined through realtime PCR. Quantification of full length genes was performed using the Applied Biosystems 7500 (Thermo Fisher Scientific Inc.) with the qScript™ One-Step SYBR® Green qRT-PCR Kit, ROX™ (QuantaBio Inc.) and oligonucleotide primers (Prim_SEQ1_for [ATGCCCTTGTCCAGTC], Prim_SEQ1_rev[CTAGAAAGCACAGCTG]) annealing (53°C) at the ends of the full length gene. The use of 1 million oligonucleotide molecules or more resulted in easily detectable amounts of full length oligonucleotide of SEQ ID NO: 1 after passing through the *in vitro* digestion assay (crossing point (C_{P}) after 23 of 40 cycles). In order to simulate the *in vitro* passage of a traditional extract, a piece of dried beef meat ("beef jerky", *Bos taurus)* was used and oligonucleotides for the corresponding growth factor 1 (GenBank: V00111.1) derived (for [acggctcagg gtccgtgaca], rev [ctaataaaat gaggaaattg]). Dried beef samples were rinsed in deionized, sterile water for 14 hours at 21°C followed by an incubation with proteinase K (400 ng/mL, P2308 Sigma-Aldrich, USA) and guanidine hydrochloride (5 mol/L, CAS Number: 50-01-1) in 1 mL volume for 12 h at 55ºC. The sample was spun down applying centrifugation (15,000 g for 10 min), followed by a phenol/chloroform extraction. Only small amounts of the growth factor 1 gene oligonucleotide could be detected before *in vitro* digestion (C_{P} at cycle 35 of 40) and no signal above the background threshold after *in vitro* digestion, clearly underlining the superiority of the synthetic oligonucleotide.

### Example 5

### Effect of stabilizers on oligonucleotide stability

*In vitro* digestion of oligonucleotides was performed as described in Example 4 with oligonucleotides alone and in combination with various stabilizers. Detection after simulated digested passage at various stages (oral, gastric, intestinal) was again performed by realtime PCR under the conditions indicated in Example 4. Various stabilizers were tested: Stabi1:potassium alginate; Stabi2:hyaluronic acid; Stabi3:poly(ethylene-co-vinyl acetate) Stabi4:poly-L-lactide. As can be seen from Figure 1, stabilizers had an advantageous effect during a simulated digestive passage since the percentage of detected oligonucleotide (in relation to the initial amount of oligonucleotide) was higher with stabilizer than without stabilizer at all time points.

## Claims

1. A formulation comprising at least 1 million oligonucleotides of mammalian non-human DNA, which oligonucleotides have at least 80% homology to one or more of the sequences selected from the group consisting of SEQ ID NOs: 1 to 3 for administration to humans.

2. A formulation according to claim 1, wherein the oligonucleotides are present in the form of DNA-carrier complexes comprising oligonucleotides and a suitable physiologically acceptable stabilizer.

3. A formulation according to claim 1 or 2, wherein the formulation is a cosmetic formulation or a topical dermatological formulation.

4. A formulation according to claim 1 or 2, wherein the formulation is a food formulation or a beverage food formulation.

5. A formulation according to claim 1 or 2, wherein the formulation is a medicinal formulation.

6. A method for producing the formulation according to any one of claims 1 to 3, comprising the steps of
(i) providing an oligonucleotide selected from the group consisting of SEQ ID NOs: 1 to 3 in a lyophilized form;
(ii) rehydrating the oligonucleotide in lyophilized form.

7. A method of claim 6, additionally comprising a step of mixing the formulation with cosmetic or food products.

8. Non-therapeutic use of the formulation of any one of claims 1 to 5 in a personal lubricant or a spice mixture.

9. A kit comprising a formulation of any one of claims 1 to 5.

10. A formulation comprising at least 1 million molecules of a mammalian non-human polypeptide, which polypeptide is encoded by a sequence that is at least 80% homologous to one or more of the sequences selected from the group consisting of SEQ ID NOs: 1 to 3 for administration to humans.

## Patentansprüche

1. Eine Formulierung, die mindestens 1 Million Oligonukleotide von nicht menschlicher Säuger-DNA umfasst, wobei die Oligonukleotide mindestens 80 % Homologie zu einer oder mehreren Sequenzen, aus der Gruppe SEQ ID NOs: 1 bis 3 ausgewählt werden, zur Verabreichung an Menschen.

2. Formulierung nach Anspruch 1, wobei die Oligonukleotide in Form von DNA-Trägerkomplexen vorliegen, die Oligonukleotide und einen geeigneten, physiologisch verträglichen Stabilisator umfassen.

3. Formulierung nach Anspruch 1 oder 2, wobei die Formulierung eine kosmetische Formulierung oder eine topische dermatologische Formulierung ist.

4. Formulierung nach Anspruch 1 oder 2, wobei die Formulierung eine Lebensmittelformulierung oder eine Getränke-Lebensmittelformulierung ist.

5. Formulierung nach Anspruch 1 oder 2, wobei die Formulierung eine medizinische Formulierung ist.

6. Verfahren zur Herstellung der Formulierung nach einem der Ansprüche 1 bis 3, umfassend die Schritte von
(i) Bereitstellen eines Oligonukleotids, ausgewählt aus der Gruppe bestehend aus SEQ ID NOs: 1 bis 3 in lyophilisierter Form;
(ii) Rehydratisieren des Oligonukleotids in lyophilisierter Form.

7. Verfahren nach Anspruch 6, das zusätzlich einen Schritt des Mischens der Formulierung mit Kosmetik- oder Lebensmittelprodukten umfasst.

8. Nicht-therapeutisch Verwendung der Formulierung eines der Ansprüche 1 bis 5 in einem persönlichen Gleitmittel oder einer Gewürzmischung.

9. Kit, umfassend eine Formulierung nach einem der Ansprüche 1 bis 5

10. Eine Formulierung, die mindestens 1 Million Moleküle eines nicht-menschlichen Säuger-Polypeptids umfasst, wobei das Polypeptid von einer Sequenz codiert wird, die zu mindestens 80 % Homologie zu einer oder mehreren Sequenzen, aus der Gruppe SEQ ID NOs: 1 bis 3 ausgewählt werden, zur Verabreichung an Menschen.

## Revendications

1. Formulation comprenant au moins 1 million d'oligonucléotides d'ADN d'un mammifère non-humain, lesquels oligonucléotides ont au moins 80 % d'homologie avec une ou plusieurs des séquences choisies dans le groupe constitué des SEQ ID NO: 1 à 3 pour l'administration à l'homme.

2. Formulation selon la revendication 1, dans laquelle les oligonucléotides sont présents sous forme de complexes ADN-support comprenant des oligonucléotides et un stabilisant approprié physiologiquement acceptable.

3. Formulation selon la revendication 1 ou 2, dans laquelle la formulation est une formulation cosmétique ou une formulation dermatologique topique.

4. Formulation selon la revendication 1 ou 2, dans laquelle la formulation est une formulation alimentaire ou une formulation alimentaire-boisson.

5. Formulation selon la revendication 1 ou 2, dans laquelle la formulation est une formulation médicinale.

6. Procédé de production de la formulation selon l'une quelconque des revendications 1 à 5, comprenant les étapes de
(i) fournir un oligonucléotide choisi dans le groupe constitué des SEQ ID NO: 1 à 3 sous une forme lyophilisée;
(ii) réhydrater l'oligonucléotide sous forme lyophilisée.

7. Procédé selon la revendication 6, comprenant en outre une étape de mélange de la formulation avec des produits cosmétiques ou alimentaires.

8. Utilisation de la formulation selon l'une quelconque des revendications 1 à 5 dans un lubrifiant personnel ou un mélange d'épices.

9. Kit comprenant une formulation selon l'une quelconque des revendications 1 à 5.

10. Formulation comprenant au moins 1 million de molécules d'un polypeptide d'un mammifère non-humain, lequel polypeptide est codé par une séquence qui est au moins à 80 % homologue à une ou plusieurs des séquences choisies dans le groupe constitué des SEQ ID NO: 1 à 3 pour l'administration à l'homme.
